(19) Europäisches Patentamt
European Patent Office
Office européen des brevets

(11) Veröffentlichungsnummer: **0 015 473**
**A1**

# (12) EUROPÄISCHE PATENTANMELDUNG

(21) Anmeldenummer: **80100928.3**

(22) Anmeldetag: **25.02.80**

(51) Int. Cl.³: **C 12 N 11/08**, G 01 N 33/54

(30) Priorität: **28.02.79 EP 79810019**

(43) Veröffentlichungstag der Anmeldung: **17.09.80 Patentblatt 80/19**

(84) Benannte Vertragsstaaten: **CH DE FR GB IT NL**

(71) Anmelder: **F.HOFFMANN-LA ROCHE & CO. Aktiengesellschaft, CH-4002 Basel (CH)**

(72) Erfinder: **Heusser, Christoph, Dr., Im Niederholzboden 15, CH-4125 Riehen (CH)**
Erfinder: **Stocker, John, Dr., Gstaltenrainweg 67, CH-4125 Riehen (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

## (54) Verfahren zum Immobilisieren von Zellen.

(57) Es wird eine Methode zum Immobilisieren von Zellen an eine Fläche beschrieben, bei der die Zellen mit Hilfe eines Kupplungsmittels an die Oberfläche eines Plastikmaterials, z.B. Polyvinylchlorid oder Polystyrol, gebunden werden. Das Plastikmaterial wird vorzugsweise in Form einer Mikrotiterplatte verwendet. Als Kupplungsmittel wird vorzugsweise ein Aldehyd mit mindestens 2 Aldehydgruppen, insbesondere Glutaraldehyd verwendet. Die erwähnte Methode zum Immobilisieren von Zellen eignet sich besonders zur Bestimmung der Bindung von Antikörpern.

EP 0 015 473 A1

25. Feb. 1980

0015473



RAN 4090/111

F. Hoffmann-La Roche & Co. Aktiengesellschaft, Basel, Schweiz

Verfahren zum Immobilisieren von Zellen

Die vorliegende Erfindung betrifft ein Verfahren zum Immobilisieren von Zellen, welches bei der Bestimmung der Bindung von Antikörpern verwendbar ist.

Das Auffinden von Antikörpern gegen Oberflächenantigene von Zellen verlangt ein bequemes, reproduzierbares Nachweissystem. Nachweismethoden, bei denen die Bindung von Antikörpern gemessen werden, haben einen Vorteil gegenüber Nachweismethoden, bei denen eine Effektorfunktion des gebundenen Antikörpers (Hämagglutination, Zytotoxizität) untersucht wird. Mit Hilfe von Bindungsnachweisen können Antikörper aller Immunglobulinklassen aufgespürt werden. Im Weiteren können diese Nachweise zur Aufspürung von Antigenen auf der Oberfläche von diversen Zelltypen angewendet werden. Die neuliche Entwicklung von Zellfusionstechniken, welche die Herstellung von monoklonalen Antikörpern gegen Oberflächenantigene von Zellen erlauben, hat das Bedürfnis nach einem raschen, gross angelegten Screeningtest für die Be-

Klt/1.2.80

stimmung solcher Antikörper im Ueberstand von Gewebskulturen hervorgerufen. Eine Methode, die jetzt verwendet wird, ist der Radioimmunoassay (RIA), bei dem die Bindung von Antikörpern an frische oder Glutaraldehyd fixierte Zellen in Suspension gemessen wird. Durch die Zentrifugation und Resuspension der Zellen während der Waschvorgänge ist diese Nachweismethode jedoch zeitaufwendig.

Die vorliegende Erfindung bezieht sich nun auf ein Verfahren zum Immobilisieren von Zellen biologischer Natur an einer Fläche, welches dadurch gekennzeichnet ist, dass man diese Zellen mit Hilfe eines Kupplungsmittels an die Oberfläche eines Plastikmaterials bindet.

Gemäss dem Verfahren der vorliegenden Erfindung bleiben die Zellen während des Waschens und der Inkubation an der Oberfläche des Plastikmaterials angeheftet. Die Zellen behalten ihre Oberflächenantigene, welche allo- und xenogenische Antikörper binden und in Radioimmunoassay oder anderen Bestimmungsmethoden, wie Immunofluoreszenz, Enzymimmunoassay oder bei Bestimmungen mit anderen geeigneten Markierungsmitteln verwendet werden können. Zum Waschen der Zellen müssen die Platten lediglich in Pufferlösung getaucht werden, worauf überflüssige Pufferlösung weggeschüttet wird.

Das Verfahren gemäss vorliegender Erfindung eignet sich besonders zur Immobilisierung von Blutzellen, z.B. Lymphozyten, Erythrozyten, Thrombozyten oder Granulozyten.

Andererseits können gemäss dem Verfahren gemäss vorliegender Erfindung Gewebskulturzellen, Tumorzellen oder transformierte Zellen immobilisiert werden.

Nach einem weiteren Aspekt der vorliegenden Erfindung können Organzellen, z.B. normale oder pathologische Zellen, wie Milz- oder Thymuszellen, immobilisiert werden.

Nach einem weiteren Aspekt der vorliegenden Erfindung können Gewebskulturzellen, wie Fibroblastzellen, immobilisiert werden.

Das Kupplungsmittel, das gemäss vorliegender Erfindung zur Bindung der Zellen an die Oberfläche des Plastikmaterials verwendet wird, ist bevorzugt ein Aldehyd.

Bevorzugte Aldehyde sind solche, welche mindestens zwei Aldehydgruppen aufweisen. Beispiele solcher Aldehyde entsprechen der allgemeinen Formel

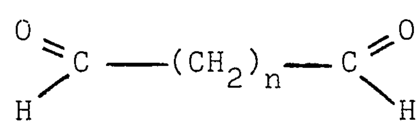

I

worin n eine ganze Zahl von 0 bis 20 darstellt.

Ein ganz besonders bevorzugter Aldehyd ist Glutaraldehyd.

Alle Arten von Plastikmaterialien können gemäss vorliegender Erfindung verwendet werden. Besonders geeignet sind Polyvinylchlorid und Polystyrol. Als weiteres Beispiel eines solchen Plastikmaterials sei Plexiglas erwähnt.

Nach einem bevorzugten Aspekt der vorliegenden Erfindung wird das Plastikmaterial, z.B. das Polyvinylchlorid oder das Polystyrol, in Form einer Microtiterplatte oder eines Röhrchens verwendet.

Nach einem weiteren Aspekt der vorliegenden Erfindung werden die Zellen an eine Oberfläche gebunden, welche mit Antikörpern beladen ist, die mit den Zellen reagieren. Gemäss diesem Aspekt dienen die Antikörper als eine Zwischenschicht, welche das Binden der Zellen an das Plastikmaterial zustande bringt.

## Beispiel 1

### Materialien und Methoden

#### Zellen

Mausthymozyten und Milzzellen werden aus 5 Wochen alten C57BL/6 und DBA/2 Mäusen gewonnen. Die Entfernung der Erythrozyten von der Milzzellensuspension erfolgt mit Hilfe eines Ammoniumchlorid enthaltenden Puffers. Menschliche Lymphozyten werden aus heparinisiertem Blut gewonnen.

Schaferythrozyten werden vor der Verwendung 4 Wochen in Alsever's Lösung gelagert.

#### Antiseren

a) Mausalloantikörper. Ascites Antikörper gegen den jeweils anderen Zellstamm werden in DBA/2 und C57BL/6 Mäusen erzeugt. Ein Gemisch von $5 \times 10^7$ Milz- und Thymuszellen wird verwendet, und jede Maus erhält über 7 Tage verteilt insgesamt 4 Injektionen. Die ersten zwei Injektionen erfolgen intravenös und die weiteren intraperitoneal. Durch i.p. Injektion von 1 ml Freund's Adjuvans wird Ascites erzeugt.

b) Kaninchen Antiserum gegen Mausimmunglobulin (RaMIg). Kaninchen werden mit Maus IgG immunisiert, welches aus Mausserum mittels Staphylococcal Protein A-Sepharose gewonnen wurde.

Die Kaninchen erhalten 0,5 mg Maus IgG in komplettem Freund's Adjuvans an verschiedenen Stellen subkutan und erhalten 4 Wochen später Boosterinjektionen von 0,5 mg Maus IgG in Freund's inkompletten Adjuvans. 1 Woche nach der Boosterinjektion wird das Serum entnommen, welches während 30 Minuten bei 56°C hitzeinaktiviert wurde.

Monoklonale Antikörper

Antikörper gegen menschliches $\beta_2$-Mikroglobulin, menschliche B-Zellen Antigene und gegen einen nicht-polymorphen Determinanten auf dem menschlichen HLA Komplex werden mit N 26/114, N 5/1 bzw. N 34 bezeichnet. Diese Antikörper werden gemäss dem Verfahren von Trucco et al. in Nature 273, 666 (1978) hergestellt.

Monoklonale Antischaferythrozyten Antikörper, Antitrinitro-phenyl (TNP) Antikörper und überständiges Medium der Mausmyelomlinie X63 werden nach der Methode von G. Köhler und C. Milstein in Eur.J.Immunol. 6, 511 (1976) hergestellt.

Iodierung der spezifischen Antikörper

RaMIg wird iodiert, wobei es zum Schutz der Bindungsstellen der Antikörper an Maus Ig-gekuppelte Sepharose gebunden ist. Die markierten Antikörper

($[^{125}I]$RaMIg) werden mit 0,2 M Glycinhydrochlorid vom pH 2,3 enthaltend Trägerprotein eluiert.

Versuchsplatten

Es werden flexible Polyvinylchlorid Mikrotiterplatten mit U oder V Vertiefungen von Cooke Engineering Co., CA, U.S.A. (Nr. 220-24 und 220-25) verwendet. Zur Herstellung eines stabilen Zellmonolayer sind die Platten mit den U Vertiefungen besser geeignet (vgl. Resultate).

Glutaraldehydmethode zur Immobilisierung der Zellen an den Platten

Zellen werden 3-Mal mit phosphatgepufferter Kochsalzlösung vom pH 7,2 gewaschen. Zur Verteilung auf der Platte werden die Leukozyten auf $2,0x10^7$/ml resuspendiert und die Erythrozyten auf 0,2% in phosphatgepufferter Kochsalzlösung. 50 µl Zellsuspension werden in die Vertiefungen gegeben, mit Ausnahme von Kontrollvertiefungen, welche ein Mass für die nicht-spezifische Bindung der serologischen Reagenzien an das Plastikmaterial liefern. Die Platten werden während 5 Minuten bei 50-100 X g zentrifugiert. Ohne die Zellschichten zu zerstören, werden die Platten bei 4°C in ein 1 l Becherglas getaucht, welches eine frisch zubereitete 0,25%ige Lösung von Glutaraldehyd in phosphatgepufferter Kochsalzlösung enthält. Zur Vermeidung der Zerstörung der

Zellschicht, sowie von Lufteinschlüssen in den Vertiefungen wird das Eintauchen sehr vorsichtig durchgeführt. Nach 5 Minuten wird die Platte entfernt und die Glutaraldehydlösung wird durch vorsichtiges Abschütteln verworfen. Durch aufeinanderfolgendes 3maliges Eintauchen der Platte in Glasbecher enthaltend phosphatgepufferte Kochsalzlösung und jeweiliges Abschütteln der Platte wird freier Glutaraldehyd weggewaschen. Die Platte wird dann in einen Becher getaucht, welcher phosphatgepufferte Kochsalzlösung mit 1% Rinderserumalbumin, 1% normal Kaninchenserum und 0,2% Natriumazid (RIA Puffer) enthält. Zur Sättigung der Proteinbindungsstellen auf dem Plastik wird die Lösung während einer Stunde bei Raumtemperatur stehengelassen. Nach dieser Zeit wird die Platte entweder sofort benutzt oder bei 4°C ohne Aenderung des Puffers gelagert.

Radioimmunverfahren unter Verwendung von Zellen die an Plastikmaterial gebunden sind

Der RIA Puffer wird von der Platte gegossen, und es werden 50 µl von Verdünnungen der Antiseren oder von überständigem Medium enthaltend monoklonale Antikörper zugegeben. Die Platte wird während einer Stunde bei Raumtemperatur stehen gelassen. Darauf wird die Platte wie vorher erwähnt durch nachfolgendes Eintauchen in phosphatgepufferte Kochsalzlösung gewaschen. Zu jeder Vertiefung werden dann 25 µl RIA Puffer enthaltend $2\text{-}4 \times 10^4$ cpm [$^{125}$I]RaMIg gegeben, worauf die Platte während einer weiteren Stunde bei Raumtemperatur gehalten wird.

Zuerst wird der grosse Teil des ungebundenen radioaktiven Materials entfernt und dann der Rest durch Waschen wie oben beschrieben. Es werden 3 Tropfen phosphatgepufferte Kochsalzlösung zu jeder Vertiefung gegeben, und die Lösung wird abgesaugt oder durch Papiertücher, welche durch Aluminiumfolien geschützt sind, absorbiert. Nach 4 Waschvorgängen werden die Platten getrocknet und dann mit einem heissen Draht geschnitten. Während des Schneidens werden die Platten durch eine starre, Polystyrol Mikrotiterplatte (Cooke Engineering Co., CA, U.S.A., No. 220M-25 AR) gehalten. Zur Zählung der Radioaktivität werden die Vertiefungen in Röhrchen gegeben.

Resultate

Haftung der Zellen an der Plastikplatte

Verschiedene Mengen von Mausmilzzellen entweder in Form der ursprünglichen Suspension oder nach Entfernung der Erythrozyten werden in die Vertiefungen von V oder U Mikrotiterplatten gegeben. Das Immobilisieren und Waschen erfolgt wie früher angegeben. Eine Untersuchung mit dem Präpariermikroskop ergibt, dass ein Zellmonolayer entsteht, wenn $1\text{-}2 \times 10^6$ Milzzellen zur U-Platte gegeben werden. Im Fall der V-Platte oder auch im Fall der U-Platte wenn eine grössere Anzahl von Zellen zugegeben werden, tendieren die Zellen zu einem Kügelchen zusammenzuballen,

welches gelegentlich während des Waschens von der Platte gehoben wird. In den nachfolgenden Experimenten wurden U-Platten verwendet.

Zu den so auf der Platte fixierten Zellen wird [$^{125}I$] RaMIg gegeben, um ein Mass zu liefern für die Anzahl der gebundenen Zellen, welche Oberflächen Ig aufweisen. Nach einer Stunde stehenlassen bei Raumtemperatur werden die Platten gewaschen, worauf für jede Vertiefung die Radioaktivität gezählt wird. Wie man der Tabelle 1 entnehmen kann, zeigte die an die Zellen gebundene Radioaktivität ein Maximum bei $1\text{-}2 \times 10^6$ zugegebener Milzzellen. Der Grad der Bindung war für die beiden verwendeten Zellpräparate ähnlich. Es konnte so gezeigt werden, dass die Anwesenheit von Erythrozyten mit der Bindung des radioaktiv markierten Antikörpers an die Lymphozyten in der Zellschicht nicht interferiert.

Oberflächenantigene von Mauszellen, die an eine Platte gebunden sind

Es wird die Reaktion von Alloantiseren gegen Oberflächenantigene von Zellen überprüft, die mittels Glutaraldehyd an die Platte gebunden sind. Es werden Thymozyten verwendet, denn diese Zellpopulation enthält wenig Zellen mit oberflächlichem lg. Damit kann ein direktes Binden von [$^{125}I$] RaMIg an die Zellen auf ein Minimun gebracht werden. Zu jeder Vertiefung werden $1,0 \times 10^6$ Thymozyten von DBA/2 oder

C57BL/6 Mäusen zugegeben und mit Glutaraldehyd fixiert. 50 $\mu l$ von Verdünnungen von entweder Alloantiseren C57BL Anti-DBA oder DBA Anti-C57BL werden zugegeben, worauf man die Platten eine Stunde stehen lässt und dann wäscht. Hierauf wird $[^{125}I]$RaMIg zugegeben. Die Ergebnisse der Bindung dieser beiden Antiseren sind in Tabelle 2 zusammengefasst. Die Spezifität dieser Antiseren für die relevanten Markerzellen wird demonstriert.

<u>Bindung von Antiseren zu fixierten und unfixierten Zellen</u>

Die Resultate der Bindung der Alloantiseren zu Zellen, welche an die Platte angeheftet sind, werden verglichen mit den Resultaten der Bindung zu unfixierten Thymozyten und zu in Suspension fixierten Thymozyten. Wie in Tabelle 3 gezeigt wird, haben die an Plastik gebundenen Zellen ein gleich hohes Bindungsvermögen für die Alloantiseren wie die fixierten oder unfixierten Zellen in der Suspension. Da die selbe Zahl von Zellen ($1{,}0 \times 10^6$) für jede Bestimmung verwendet wird zeigt dies Resultat an, dass die Glutaraldehyd-Bindungsmethode die Bindung und Beibehaltung aller auf die Platte gegebener Zellen erlaubt. Das spezifische Signal beurteilt durch die Differenz zwischen Bindung an relevante und irrelevante Markerzellen ist für die an die Platte angehefteten Zellen äusserst ausgeprägt. Im Weiteren kann gezeigt werden, dass Zellen, welche mittels Glutaraldehyd an die Platte gebunden sind und während 7 Wochen vor der Bestimmung bei $4^{o}C$ in RIA-Puffer gelagert wurden, eine ähnliche Bindungskapazität aufweisen wie diejenigen, die am Tag der Bestimmung auf die Platte gebunden werden (diese Daten sind nicht gezeigt).

Verwendung von an die Platte angehefteten Zellen zur Auffindung monoklonaler Antikörper

Ueberständiges Medium von Kulturen, welche monoklonale Mausantikörper produzieren, werden bezüglich ihrer Bindung an Zellen, welche an die Platte angeheftet sind, überprüft. Diese Antikörper von bekannten Ig-Subklassen haben eine Spezifität für definierte Oberflächenantigene von Zellen. Es werden Zellschichten menschlicher peripherer Blutlymphozyten (PBL) und rote Blutzellen von Schafen (SRBC) verwendet. 50 $\mu$l von überständigem Medium von Hybridzellkulturen werden zugegeben und die Bindung zu den zwei Zelltypen wird untersucht. Aus Tabelle 4 geht hervor, dass die Antikörper auf den relevanten Markerzellen gefunden werden konnten. Im Falle von N 5/1, einem Antikörper, welcher gegen menschliche B-Zellen Oberflächenantigene gerichtet ist, ist die Bindung 5mal geringer als die Bindung von Anti-$\beta_2$-Mikroglobulin. Dies entspricht dem Vorherrschen der B-Zellen innerhalb von PBL.

Tabelle 1

BINDUNG VON $^{125}$I MARKIERTEM ANTI-MAUS Ig ZU MILZZELLEN, WELCHE AN DER PLATTE ANGEHEFTET SIND [a]

| Zell Nr. ($x10^{-6}$) | Zugegebene Zellen: Ganze Milzzellen | Zugegebene Zellen: Milzzellen, bei denen die Erythrozyten entfernt wurden |
|---|---|---|
| 0.25 | 2954 ± 283 [b] | 2785 ± 174 |
| 0.5 | 4289 ± 279 | 4325 ± 259 |
| 1.0 | 5184 ± 266 | 5109 ± 76 |
| 2.0 | 5475 ± 660 | 6130 ± 281 |
| 4.0 | 4157 ± 194 | 5212 ± 217 |
| Zell Nr. | 640 ± 218 | |

[a] Es werden verschiedene Mengen von ganzen Milzzellen oder von erythrozytenlosen Milzzellen auf die Platte gegeben zur Glutaraldehydfixierung. Die direkte Bindung von [$^{125}$I]RaMIg wird gemessen.

[b] cpm. Mittel von 4 wiederholten Bestimmungen ± Standarderror für den Durchschnitt (S.E.M.).

Tabelle 2

REAKTION VON ALLOANTISEREN MIT ZELLEN, WELCHE MITTELS GLUTARALDEHYD AN DER PLATTE ANGEHEFTET SIND [a]

| Alloantiserum | Verdünnung | Thymocyten DBA/2 | Anzahl der Zellen | Thymocyten C57BL/6 |
|---|---|---|---|---|
| C57BL/6 | 1/100 | 3161 ± 148 [b] | 275 ± 23 | 638 ± 27 |
| Anti-DBA/2 | 1/1000 | 1494 ± 19 | 158 ± 11 | 208 ± 18 |
| Kein Antiserum | | 144 ± 10 | 76 ± 6 | 120 ± 17 |
| DBA/2 | 1/100 | 1099 ± 35 | 441 ± 53 | 2334 ± 58 |
| Anti-C57BL/6 | 1/1000 | 329 ± 12 | 226 ± 39 | 881 ± 28 |

[a] Thymocyten von DBA/2 oder C57BL/6 Mäusen werden an der Platte angeheftet und die Bindung der Alloantiseren wird unter Verwendung von [$^{125}$I]RaMIg gemessen.

[b] cpm. Durchschnitt von 4 nacheinanderfolgenden Bestimmungen ± S.E.M.

Tabelle 3

VERGLEICH DER BINDUNG VON ZELLEN, DIE AN DER PLATTE ANGEHEFTET SIND GEGENUEBER ZELLEN IN SUSPENSION [a]

In Abwesenheit von Serum ergaben die 3 Versuchstypen die folgenden nicht-spezifischen Aufnahmen: plattenfixiert, 160 $\pm$ 31; suspensionsfixiert, 117 $\pm$ 11; frische Suspension, 526 $\pm$ 376. Diese Werte waren ähnlich für die 2 Thymocytenspenderstämme.

| Thymocytenstamm | Versuchstyp [b] | | |
|---|---|---|---|
| | plattenfixiert | suspensionsfixiert | frische Suspension |
| DBA/2 | 3544 $\pm$ 353 [c] | 1264 $\pm$ 98 | 1510 $\pm$ 394 |
| C57BL/6 | 581 $\pm$ 62 | 380 $\pm$ 111 | 838 $\pm$ 196 |
| Anzahl der Zellen | 265 $\pm$ 43 | 333 $\pm$ 38 | 299 $\pm$ 69 |

[a] Die Bindung von Anti-DBA/2 (1:100) an DBA/2 oder C57BL/6 Thymocyten wurde nach 3 verschiedenen Versuchstypen bestimmt. Die Aufnahme von Anti-DBA/2 wird durch $[^{125}I]$RaMIg bestimmt.

[b] Die 3 Versuchstypen waren: Zellschichten an der Platte angeheftet mittels Glutaraldehyd (plattenfixiert); in der Suspension mit Glutaraldehyd fixierte Zellen (suspensionsfixiert) und unfixierte Zellen in der Suspension (frische Suspension).

[c] cpm. Durchschnitt von 4-8 nacheinanderfolgenden Bestimmungen $\pm$ S.E.M.

Tabelle 4

SPEZIFISCHE BINDUNG MONOKLONALER ANTIKOERPER AN ZELL-MONOLAYER [a]

| Monoklonaler Antikörper | | Bindung an Zellmonolayer | | |
|---|---|---|---|---|
| Bezeichnung | Spezifität | menschliches PBL | SRBC | Nr. der Zellen |
| N 34 | HLA-Komplex | 4279 ± 164 [b] | 201 ± 34 | 285 ± 21 |
| N 26/114 | menschliches $\beta_2$ Mikroglobulin | 5416 ± 593 | 166 ± 12 | 356 ± 13 |
| N 5/1 | menschliches B-Zellen Antigen | 1150 ± 8 | 133 ± 17 | 182 ± 13 |
| Sp 2/HL | SRBC | 323 ± 12 | 3596 ± 526 | 264 ± 22 |
| Hy 2-19 | TNP | 465 ± 53 | 383 ± 33 | 672 ± 35 |
| X63 | unbekannt | 264 ± 14 | 129 ± 8 | 142 ± 8 |
| Puffer | --------- | 180 ± 8 | 150 ± 46 | 124 ± 9 |

[a] Monoklonale Mausantikörper verschiedener Spezifitäten wurden untersucht auf ihre Bindung an Zellschichten von menschlichen peripheren Lymphocyten (PBL) oder Schaferythrocyten (SRBC), welche mittels Glutaraldehyd an die Platten gebunden waren. Die Bindung der monoklonalen Antikörper wurde durch [$^{125}$I]RaMIg bestimmt.

[b] cpm. Mittel von 4 nacheinanderfolgenden Bestimmungen ± S.E.M.

## Beispiel 2

Die erfindungsgemässe Methode zur Immobilisierung von Zellen wird angewendet, um die Anwesenheit von Antikörpern gegen definierte menschliche Erythrozytenantigene zu untersuchen. Zu diesem Zwecke werden kommerziell erhältliche Antiseren gegen klinisch wichtige Rhesusantigene auf ihre Bindungsfähigkeit gegenüber immobiliserten Erythrozyten-Zell-Schichten von verschiedenen Rhesusgruppen untersucht.

Reagentien:

Antiseren sind von der Firma Merz & Dade, Düdingen (Schweiz) und sind gegen folgende Rhesus-Determinanten gerichtete: C, E, $C^W$, CDE, c, e, CD, DE.

Menschliche Erythrozyten werden von Biotest erhalten und haben folgende Genotypen: (CCD.ee), (ccD.EE), ($C^W$cD.Ec), (ccD.ee), (Ccddee) und (ccddee).

Protein A von Staphylococcus aureus zeigt eine hohe Affinität gegenüber dem Fc-Teil von Immunoglobulin G und wird von Pharmacia (Uppsala) erhalten. Protein A wird mit $^{125}I$ radioaktiv markiert und in den folgenden Experimenten zur Bestimmung der Aufnahme von Antikörper an die Erythrozytenoberfläche verwendet.

0015473

Methode:

Die Methode ist dieselbe wie in Beispiel 1, mit der einzigen Ausnahme, dass für die Bestimmung von gebundenen Antikörpern radioaktiv markiertes Protein A verwendet wird.

Resultat:

| Rhesusphänotyp der Erythrozyten | C | c | D |
|---|---|---|---|
| (CCD,ee) | 3720 | 1260 | 3060 |
| (ccD,EE) | 540 | 7990 | 4680 |
| ($C^W$cD.Ec) | 2800 | 6510 | 3220 |
| (ccD.ee) | 1020 | 7390 | 3580 |
| (Ccddee) | 3420 | 4360 | 380 |
| (ccddEe) | 1200 | 9830 | 300 |
| (ccddee) | 1110 | 8810 | 970 |

0015473

## Patentansprüche

1. Verfahren zum Immobilisieren von Zellen biologischer Natur an eine Fläche, dadurch gekennzeichnet, dass die Zellen mit Hilfe eines Kupplungsmittels an die Oberfläche eines Plastikmaterials gebunden werden.

2. Verfahren nach Anspruch 1, dadurch gekennzeichnet, dass die Zellen Blutzellen sind.

3. Verfahren nach Anspruch 2, dadurch gekennzeichnet, dass die Blutzellen Lymphozyten oder Erythrozyten sind.

4. Verfahren nach Anspruch 2, dadurch gekennzeichnet, dass die Blutzellen Thrombozyten oder Granulozyten sind.

5. Verfahren nach Anspruch 1, dadurch gekennzeichnet, dass die Zellen Gewebskulturzellen, Tumorzellen oder transformierte Zellen sind.

6. Verfahren nach Anspruch 1, dadurch gekennzeichnet, dass die Zellen Organzellen sind.

7. Verfahren nach Anspruch 6, dadurch gekennzeichnet, dass die Organzellen normal oder pathologisch sind.

8. Verfahren nach Anspruch 7, dadurch gekennzeichnet, dass die Organzellen Milz- oder Thymuszellen sind.

9. Verfahren nach Anspruch 5, dadurch gekennzeichnet, dass die Zellen Fibroblastzellen sind.

10. Verfahren nach einem der Ansprüche 1-9, dadurch gekennzeichnet, dass das Kupplungsmittel ein Aldehyd ist.

11. Verfahren nach Anspruch 10, dadurch gekennzeichnet, dass der Aldehyd mindestens zwei Aldehydgruppen enthält.

12. Verfahren nach Anspruch 11, dadurch gekennzeichnet, dass der Aldehyd die allgemeine Formel

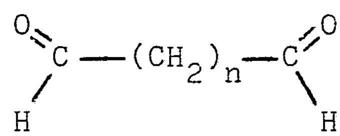

I

worin n eine ganze Zahl von 0-20 bedeutet, aufweist.

13. Verfahren nach Anspruch 12, dadurch gekennzeichnet, dass die Verbindung der Formel I Glutaraldehyd ist.

14. Verfahren nach einem der Ansprüche 1-13, dadurch gekennzeichnet, dass das Plastikmaterial Polyvinylchlorid oder Polyvinylstyrol ist.

15. Verfahren nach Anspruch 14, dadurch gekennzeichnet, dass das Plastikmaterial in Form einer Mikrotiterplatte oder eines Röhrchens vorliegt.

16. Verfahren nach Anspruch 1, dadurch gekennzeichnet, dass die Zellen an eine Oberfläche gebunden werden, welche mit Antikörpern beladen ist, die mit den Zellen reagieren.

***

Europäisches Patentamt

# EUROPÄISCHER RECHERCHENBERICHT

0015473
Nummer der Anmeldung
EP [illegible]

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | FR - A - 2 321 127 (MT. SINAI SCHOOL OF MEDICINE OF THE CITY UNIVERSITY OF NEW YORK)<br>* Seite 21, Anspruch 1a; Seite 11, Zeilen 9-35 *<br>-- | 1-3, 10-16 |
| X | CHEMICAL ABSTRACTS, Band 83, Nr. 3, 21. Juli 1975, Seite 358, Nr. 26137p<br>Columbus, Ohio, U.S.A.<br>S.Y. WONG et al.: "Immobilization of lymphocytes at surfaces by lectins"<br>& EXP. CELL RES. 1975, 92(2), 428-34<br>* Zusammenfassung *<br>-- | 1,3, 14,15 |
| X | CHEMICAL ABSTRACTS, Band 88, Nr. 11, 13. März 1978, Seite 216, Nr. 70986s<br>Columbus, Ohio, U.S.A.<br>BO. MATTIASSON et al.: "An analytical flow system based on reversible immobilization of enzymes and whole cells utilizing specific lectin-glucoprotein interactions"<br>& FEBS LETT. 1978, 85(1), 119-23<br>* Zusammenfassung *<br>-- | 1-3 |
| X | US - A - 4 078 971 (B. ARKLES et al.)<br>* Spalte 6, Anspruch 12 *<br>-- | 1,6 |
| X | FR - A - 2 265 758 (PFIZER INC.)<br>* Seiten 13,14; Beispiel 21; ./. | 1,10-13 |

KLASSIFIKATION DER ANMELDUNG (Int.Cl. 3)

C 12 N 11/08
G 01 N 33/54

RECHERCHIERTE SACHGEBIETE (Int. Cl. 3)

G 01 N 33/80
C 12 N 11/00
11/02
11/06
11/08

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 30-05-1980 | WALLINDER |



BAD ORIGINAL

Europäisches Patentamt

# EUROPÄISCHER RECHERCHENBERICHT

0015473

Nummer der Anmeldung

EP 80 10 0928



| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | Seite 15; Ansprüche 1,5,11 * | | |
| | -- | | |
| X | FR - A - 2 332 287 (BEHRINGWERKE AKTIENGESELLSCHAFT)<br>* Seite 17, Ansprüche 1,3; Seite 7, Zeilen 4,5 * | 1,2,10-13 | |
| | -- | | |
| A | DE - A - 1 806 418 (MILES LABORATORIES)<br>* Seiten 24,25; Ansprüche 1,2, 5 * | 1,2 | RECHERCHIERTE SACHGEBIETE (Int. Cl. 3) |
| | -- | | |
| E | GB - A - 2 004 892 (E.A. FISCHER)<br>* Seite 3, Zeilen 36-43; Seite 7, Anspruch 34 *<br>& FR - A - 2 410 012<br>& DE - A - 2 840 768<br>& NL - A - 78 09205<br>& EP - A - 0 001 223 | 1-4 | |
| | -- | | |
| E | GB - A - 2 005 275 (E.A. FISCHER)<br>* Seite 2, Zeilen 119-125; Seite 5, Anspruch 1 *<br>& FR - A - 2 403 386<br>& DE - A - 2 840 767<br>& NL - A - 78 09332<br>& EP - A - 0 001 224 | 1-4 | |
| | ---- | | |